# EUROPÄISCHE PATENTSCHRIFT

(11) **EP 0 917 871 B1**
(45) Veröffentlichungstag und Bekanntmachung des Hinweises auf die Patenterteilung: **28.06.2006**
(21) Anmeldenummer: 98119590.2
(22) Anmeldetag: 16.10.1998
(51) Int. Cl.: A61K 8/67, C07D 307/62, A61K 31/375, A61Q 19/00

(54) **Ascorbinsäurederivate enthaltende kosmetische und pharmazeutische Zubereitungen**
Cosmetic and pharmaceutical compositions comprising ascorbic acid derivatives
Compositions cosmétiques et pharmaceutiques contenant des dérivés de l'acide ascorbique

(30) Priorität: 14.11.1997 DE 19750526
(43) Veröffentlichungstag der Anmeldung: 26.05.1999
(73) Patentinhaber: BASF Aktiengesellschaft, 67056 Ludwigshafen (DE)
(72) Erfinder: Streicher, Harald Dr., 67069 Ludwigshafen (DE); Ostersehlt, Bernd Dr., 67133 Maxdorf (DE); Westenfelder, Horst, 67435 Neustadt (DE)

(56) Entgegenhaltungen:
- US-A- 2 980 702
- DATABASE CA [Online] CHEMICAL ABSTRACTS SERVICE, COLUMBUS, OHIO, US; TANAKA, HIROYOSHI ET AL: "Pharmaceutical studies on ascorbic acid derivatives. I. Syntheses of esters of ascorbic acid and their physicochemical properties" retrieved from STN Database accession no. 65:29695 XP002235880 -& YAKUGAKU ZASSHI (1966), 86(5), 376-83, XP002236531
- DATABASE CA [Online] CHEMICAL ABSTRACTS SERVICE, COLUMBUS, OHIO, US; INAGAKI, CHOTEN ET AL: "Metabolism of fatty acid esters of L-ascorbic acid. III. Biochemical activity of various L-ascorbic acid derivatives" retrieved from STN Database accession no. 68:66890 XP002235881 -& BITAMIN (1968), 37(2), 152-8 , XP002236532
- DATABASE CA [Online] CHEMICAL ABSTRACTS SERVICE, COLUMBUS, OHIO, US; TANAKA, HIROYOSHI ET AL: "5,6-Isopropylideneascorbic acid derivatives" retrieved from STN Database accession no. 73:99173 XP002235882 & JP 45 011690 B (SHIONOGI AND CO., LTD.) 27. April 1970 (1970-04-27)
- DATABASE CA [Online] CHEMICAL ABSTRACTS SERVICE, COLUMBUS, OHIO, US; MURAKAMI, MASUO ET AL: "5,6-0-Carbonylascorbic acid derivatives" retrieved from STN Database accession no. 74:88266 XP002235883 & JP 45 031661 B (YAMANOUCHI PHARMACEUTICAL CO., LTD.) 13. Oktober 1970 (1970-10-13)

## Beschreibung

Ascorbinsäure (Vitamin C) ist ein Antioxidans, welches in der Kosmetik breit eingesetzt wird. Es wirkt zusammen mit Vitamin E der Oxidation von ungesättigten Fettsäuren entgegen und verhindert so auch eine Lipoperoxidbildung (O. Isler, G. Brubacher, S. Ghisla, B. Kräutler eds.; Vitamine II, G. Thieme Verlag, Stuttgart, N. Y., 431, 1988). Als Vitamin ist die Ascorbinsäure auch am Metabolismus beteiligt, so z.B. bei der Hydroxylierung von Prolin in der Collagen Synthese (J. J. Burns, J. M. Rivers, L. J. Machin eds.; Third Conference on vitamin C, Ann. N. Y. Acad. Sci., 498 (1987) 1-533).

Aufgrund der zu geringen Stabilität von Ascorbinsäure in kosmetischen Formulierungen, ist es häufig erforderlich, stabilisierte Derivate der Ascorbinsäure einzusetzen. Beispiele hierfür sind Natrium-L-ascorbinsäure-monophosphat (JP 07082127, JP 05331020), L-Ascorbinsäure-2-0-D-glucosid (T. Sakamoto et al.; 19th IFSCC Congress, Sydney, 1996, Vol. 2, Paper No. 14) sowie 5,6-Isopropyliden-L-ascorbinsäure-2-phosphat (JP 08269074).

Pulikationen zur Herstellung und Analyse von Ascorbinsäurederivate und deren Verwendung als pharmazeutische Wirkstoffe wurden bereits in den sechziger Jahren des vergangenen Jahrhunderts veröffentlicht. So wurde z.B. die Herstellung von Ascorbinsäurederivaten und deren physicochemischen Eigenschaften beschrieben (Tanaka et al., Pharmaceutical studies on ascorbic derivatives. I. Syntheses of esters of ascorbic acid and their physicochemical properties. Yakugaku Zasshi (1966), 86 (5), 376-83.) und die Wirkung verschiedener Ascorbinsäurederivate als Antiscorbutmittel in Fütterrungsversuchen an Meerschweinchen untersucht (Ignaki et al. Metabolism of fatty acid esters of L-ascorbic acid. III : Biochemical activity of various L-ascorbic acid derivatives. Bitamin (1968), 37(2), 152-158.)

Die oben genannten stabilisierten Ascorbinsäurederivate heben jedoch häufig den Nachteil, daß sie in kosmetischen oder pharmazeutischen ölen zu schlecht löslich sind.

Es war daher Aufgabe der Erfindung, stabile Ascorbinsäurederivate für kosmetische Zubereitungen bereit zu stellen, die die oben genannten Nachteile nicht zeigen.

Diese Aufgabe wurde erfindungsgemäß gelöst durch Verwendung von Ascorbinsäurederivate der allgemeinen Formel I, 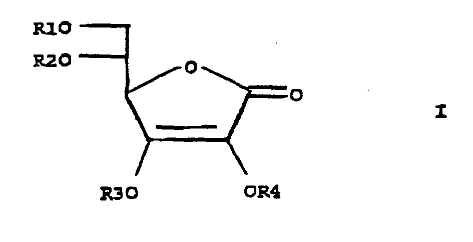 in der die variablen unabhängig voneinander folgende Bedeutung haben:
- R¹: Wasserstoff, C₁-C₂₀-Acyl, C₁-C₆-Alkoxycarbonyl;
- R²: Wasserstoff, C₁-C₂₀-Acyl, C₁-C₆-Alkoxycarbonyl,
wobei R¹ kein C₁-C₆-Alkoxycarbonyl-Rest sein darf, wenn R² für Wasserstoff steht;
- R³: Wasserstoff oder ein Kation, ausgewählt aus der Gruppe, bestehend aus NH₄⁺, Alkali- und Erdalkalimetallkationen;
- R⁴: C₁-C₆-Alkoxycarbonyl,
für kosmetische Zubereitungen.

Bei den erfindungsgemäßen Ascorbinsäurederivaten der Formel I sind als Acylreste für R¹ und R² verzweigte oder unverzweigte, gesättigte oder ungesättigte, gegebenenfalls mehrfach ungesättigte C₁-C₂₀-Acylketten zu verstehen.

Beispiele hierfür sind Acylreste der Ameisen-, Essig-, Propion-, n-Butter-, iso-Butter-, Sorbin-, n-Valerian-, iso-Valerian-, Capron-, Capryl-, Caprin-, Undecan-, Laurin-, Tridecan-, Myristin-, Pentadecan-, Palmitin-, Palmitolein-, Stearin-, Öl-, Linol-, Linolen-, Nonadecan- und Arachidonsäure.

Bevorzugt sind Acylreste langkettiger Fettsäuren mit C₁₀ bis C₂₀ Kohlenstoffketten, insbesondere Acylreste der Laurin-, Palmitin-, Palmitolein-, Stearin-, Öl- und Linolsäure.

Als Alkoxycarbonylreste für R¹, R² und R⁴ kommen solche in Betracht, deren Alkoxygruppe 1 bis 6 C-Atome, besonders bevorzugt 1 bis 4 C-Atome enthält.

Von den bevorzugten Resten sind beispielsweise zu nennen:

| | |
|---|---|
| Methoxycarbonyl- | Ethoxycarbonyl- |
| Isopropoxycarbonyl- | n-Propoxycarbonyl- |
| 1-Methylpropoxycarbonyl- | n-Butoxycarbonyl- |
| n-Pentoxycarbonyl- | 2-Methylpropoxycarbonyl- |
| 3-Methylbutoxycarbonyl- | 1,1-Dimethylpropoxycarbonyl- |
| 2,2-Dimethylpropoxycarbonyl- | Hexoxycarbonyl- |
| 1-Methyl-1-ethylpropoxycarbonyl- | |

Als besonders bevorzugte Alkoxycarbonylreste kommen in Betracht:

| | |
|---|---|
| Methoxycarbonyl- | Echoxycarbonyl- |
| Isopropoxycarbonyl- | n-Propoxycarbonyl- |
| 1-Methylpropoxycarbonyl- | n-Butoxycarbonyl- |

Als Kationen für R³ kommen NH₄⁺ sowie Vertreter aus der Gruppe der Alkali- und Erdalkalimetalle in Frage, bevorzugt Na, K, Li, Ca und Mg, besonders bevorzugt Na, K und Mg.

Unter dem Begriff Ascorbinsäurederivate sind sowohl Derivate der L- als auch der D-Ascorbinsäure (Isoascorbinsäure), bevorzugt der

L-Ascorbinsäure zu verstehen.

Einige Di- und Tricarbonate der Ascorbinsäure sind bereits bekannt- So beschreibt US 2,980,702 Verbindungen vom Typ 2,5,6-Tri-O-(C₁-C₃-alkoxycarbonyl)-L-ascorbinsäure und deren Verwendung als wärmeresistenten Zusatzstoff in Nahrungsmitteln, speziell in Backwaren.

JP 42020050 beschreibt die Synthese von 2,6-Di-O-(C₁-C₃₋alkoxycarbonyl)-L-ascorbinsäure als hitzestabiles Derivat der Ascorbinsäure.

Die Verwendung der oben genannten Ascorbinsäurederivate in der kosmetischen und Anwendung dagegen ist neu.

Die erfindungsgemäßen stabilen Ascorbinsäurederivate der Formel I eignen sich hervorragend als Wirkstoffe für kosmetische Zubereitungen.

So zeichnen sich die verbindungen u.a. dadurch aus, daß man durch Variation der Reste R¹, R² und R⁴ die Lipophilie der Ascorbinsäurederivate gezielt einstellen kann. Je nach Anforderung bei der Formulierung kosmetischer und Zubereitungen stehen dem Anwendungstechniker somit eine breite Palette stabiler Vitamin C-Derivate zur Verfügung. Besonders die Tricabonate und die Fettsäureester von Vitamin-C-2-monocarbonat lassen sich aufgrund ihrer guten Öllöslichkeit sehr gut in Zubereitungen wie beispielsweise Salben, Lotionen, Gelen oder Emulsionen einarbeiten.

Demgemäß sind auch Gegenstand der vorliegenden Erfindung kosmetische und pharmazeutische Zubereitungen, enthaltend eine wirksame Menge mindestens einer der verbindungen der Formel I sowie übliche kosmetische und Hilfs- und Zusatzstoffe.

Die o.g. Zubereitungen enthalten Verbindungen der Formel I in Anteilen von 0,01 bis 10 Gew.-%, vorzugsweise 0,1 bis 8 Gew.-%, besonders bevorzugt 0,5 bis 5 Gew.-%, bezogen auf die Gesamtmenge der kosmetischen Zubereitung.

Eingesetzt werden können die Ascorbinsaurederivate der Formel I u.a. in allen kosmetischen und pharmazeutischen Zubereitungen, die neben Wasser auch noch Emulgatoren, Stabilisatoren, natürliche Öle, kosmetische Öle, Fette , Wachse, Siliconöle, Siliconölderivate, Solubilisatoren, Lichtschutzmittel, Feuchthaltemittel, Wirkstoffe, Fonsistenzgeber, Gelbildner, Antioxidantien, Konservierungsmittel enthalten.

Als Emulgatoren können z-B. folgende Substanzen verwendet werden:

Polyglycerinfettsäureester, Ethoxylate von Fettsäuren, Sorbitanfettsäureester, Phosphorsäureester von Fettsäuren, Phospholipide, Glycerinmonostearat und Glycerinmonostearat selbstemulgierend.

### Unter Stabilisatoren versteht man:

Magnesium und Aluminiumsalze von Fettsäuren, Komplexbildner wie EDTA, NTA, MGDA, Antioxidantien wie BHT, BHA, alpha Tocopherol, Gallusäure und ihre Salze und Ester.

Natürliche öle sind z.B. Jojobaöl, Sonnenblumenöl, Erdnußöl, Mandelöl, Avocadoöl, Macadamianußöl, Rizinußöl, Maiskeimöl, Traubenkernöl.

Kosmetische Öle sind z.B. Isopropylester von Fettsäuren ganz besonders isopropylstearat, Isopropylpalmitat, Isopropylisostearat, Isopropylmyristat, Isopropyllaurat, Paraffinöl, Neutralöl.

Kosmetische Wirkstoffe sind z.B. Panthenol, Bisabolol, a-Tocopherol, a-Tocopherylacetat, Aloe Vera, Algenextrakt, Hyaluronsäure, Retinol und Retinylester, Phytantriol, Panthenylethylether, Ferulasäure.

Lichtschutzmittel, die alleine oder als Gemisch zusammen mit den verbindungen der Formel I verwendet werden können sind z.B.

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (oxybenzonum) | 131-57-7 |
| 5 | 2-Phenylbenzimidazol-5-Sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-bensophenon-5-sulfon-(Sulisobenzonum) und das Natriumsalz | 4065-45-6 |
| 13 | 3-(4'-Methyl)benzyliden-bornan-2-on | 36861-47-9 |
| 14 | 3-Benzylidenbornan-2-on | 15087-24-8 |
| 15 | 1-(4'-Isopropylphenyl)-3-phenylpropan-1,3-dion | 63250-25-9 |
| 16 | 4-Isopropylbenzylsalicylat | 94134-93-7 |
| 17 | 2,4,6-Trianilin-(o-carbo-2'-ethylhexyl-1-oxy)-1,3,5-triazin | 88122-99-0 |
| 18 | 3-Imidazol-4-yl-acrylsäure und ihr Ethylester | 104-98-3 |
| 19 | 2-Cyano-3,3-diphenylacrylsäureethylester | 5232-99-5 |
| 20 | 2-Cyano-3,3-diphenylacrylsäure-2'-ethylhexylester | 6197-30-4 |
| 21 | Methyl-o-aminobenzoate oder: 5-Methyl-2-(1-methylethyl)-2-aminobenzoate | 134-09-8 |
| 22 | Glyceryl p-aminobenzoat oder: 4-Aminobenzoesäure-1-glyceryl-ester | 136-44-7 |
| 23 | 2,2'-Dihydroxy-4-methoxybenzophenon (Dioxybenzone) | 131-53-3 |
| 24 | 2-Hydroxy-4-methoxy-4-methylbenzophenon (Mexonon) | 1641-17-4 |
| 25 | Triethanolamin Salicylat | 2174-16-5 |
| 26 | Dimethoxyphenylglyoxalsäure oder: 3,4-dimethoxy-phenyl-glyoxal-saures Natrium | 4732-70-01 |
| 27 | 3-(4'Sulfo)benzyliden-bornan-2-on und seine Salze | 56039-58-8 |
| 28 | 4-tert.-sutyl-4'-methoxy-dibenzaylmethan | 70356-09-1 |
| 29 | 2,2`,4,4`-Tetrahydrvxybenzophenon | 131-55-5 |

Die Ascorbinsäurederivate der Formel I sind auch für tensidiachen Formulierungen geeignet.

So können problemlos Haarspülungen, Shampoos, Schäume mit stabilem Vitamin C der Formel I hergestellt werden.

Die Kombination von anionischen mit kationischen Tensiden schränkt den Einsatz in Kosmetikprodukten nicht ein.

Gegenstand der Erfindung sind weiterhin Ascorbinoäurederivate der Formel Ia, 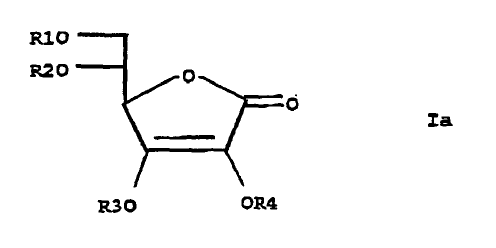 in der die Variablen unabhängig voneinander folgende Bedeutung haben:
- R¹ und R²: unabhängig voneinander
wasserstoff oder C₁-C₂₀-Acyl;
- R³: Wasserstoff oder ein Kation, ausgewählt aus der Gruppe, bestehend aus NH₄⁺, Alkal- und Erdalkalimetallkationen;
- R⁴: C₁-C₆-Alkoxycarbonyl.

Die Synthese der Ascorbinsäurederivate der Formel Ia ausgehend von dem Ausgangsprodukt IIa erfolgt analog dem Verfahren gemäß der japanischen Patentveröffentlichung JP 42020050. Bezüglich näherer Einzelheiten sei auf diese Veröffentlichung hingewiesen.

In den nachfolgenden Beispielen wird die Herstellung der erfindungsgemäßen Ascorbinsäurederivate der Formel I sowie die Zusammensetzung kosmetischer Formulierungen, die diese Ascorbinsäurederivate enthalten, näher erläutert.

### Beispiel 1

### 2,5,6-Tri-O-(isopropyloxycarbonyl)-L-ascorbinsäure

Zu 80 g (0,454 mol) L-Ascorbinsäure in 350 ml (4,3 mol) Pyridin wurden bei -15°C 1,5 L (1,5 mol) einer 1 M Lösung von Chlorameisensäureiscpropyl-ester in Toluol zugetropft, wobei Pyridiniumhydrochlorid als weißer Niederschlag ausfiel. Die resultierende Suspension wurde weitere 1,5 Stunden bei Raumtemperatur gerührt und anschließend das Pyridiniumhydrochlorid abfiltriert. Das gelbe Filtrat wurde im vakuum eingeengt und das daraus hervorgehende rötliche, ölige Rohprodukt nach Aufnahme in Dichlormethan zweimal mit 1 M HCl-Lösung gewaschen. Die organische Unterphase wurde darauf viermal mit gesättigter (10%iger) Natriumhydrogencarbonatlösung extrahiert und die vereinten wässrigen Extrakte (jeweils Oberphasen) mit konz. Salzsäure bis zur einsetzenden Trübung (ca. pH 1) versetzt. Diese wäßrige Suspension wurde anschließend dreimal mit Dichlormethan extrahiert und die vereinigten organischen Extrakte (jeweils Unterphasen) über Na₂SO₄ getrocknet und im Vakuum eingeengt. Nach Vakuumtrocknung wurde ein gelbes Wachs erhalten.
Ausbeute: 68,0 g (34,5 %)
¹³C-NMR: δ = 165,82 (CO-Ascorbyl), 154,90 (C-3-Ascorbyl), 154,06, 153,58, 153,09 (3x CO-Carbonat), 115,04 (C-2-Ascorbyl), 76,02 (C-4-Ascorbyl), 73,63, 73,25, 73,13 (3x CH(CH₃)₂) , 72, 88 (C-5-Ascorbyl), 64,44 (C-6-Ascorbyl), 21,70, 21,69, 21,56, 21,54, 21,48, 21,47 (6x CH₃) ppm.

### Beispiel 2

### 6-o-Palmitoyl-2-o-(isopropyloxycarbonyl)-L-aseorbinsäure

Zu 17,8 g (0,042 mol) 6-O-Palmitoyl-L-ascorbinsäure in 100 ml (1,24 mol) Pyridin wurden bei -15°C 45 ml (0,045 mol) einer 1 M Lösung von Chlorameisensäureisopropylester in Toluol zugetropft. Der Reaktionsansatz wurde eine weitere Stunde bei Raumtemperatur gerührt und das ausgefallene Pyridiniumhydrochlorid abfiltriert. Das gelbe Filtrat wurde unter mehrmaligem Codestillieren mit Toluol im Vakuum eingeengt.

Der braune ölige Rückstand wurde in Dichlormethan/Toluol (1:1) aufgenommen und zweimal mit 1 M HCl-Lösung gewaschen, Trocknen der organischen Phase über Na₂SO₄ und Einengen im Vakuum lieferte einen dunkelgelb bis braunen Feststoff, der in verteilter Form nach zweimaligem Suspendieren in wenig kaltem Essigester als weißer Feststoff filtriert wurde.
Auswaage: 13,3 g (61,9 %)
¹³C-NMR: δ = 174,11 (CO-Palmitoy1), 166,80 (CO-Ascorbyl), 156,62 (OCOO-Carbonat), 153,23 (C-3-Ascorbyl), 114,60 (C-2-Ascorbyl), 75,98 (C-4-Ascorbyl); 75,60 (OCH(CH₃)₂); 67,94 (C-5-Ascorbyl) ; 64,43 (C-6-Ascorbyl); 34,10 (C-2-Palmitoyl) ppm.

### Beispiel 3

### 5, 6-O-Isopropyliden-2-O-(isopropyloxycarbonyl)-L-ascorbinsäure

zu 85,3 g (0,395 mol) 5,6-O-Isopropyliden-L-ascorbinsäure in 300 ml (3,7 mol) Pyridin wurden bei -15°C 415 ml (0,415 mol) einer 1 M Lösung von Chlor-ameisensäureisopropylester in Toluol zugetropft, wobei Pyridiniumhydrochlorid als weißer Feststoff ausfiel. Es wurde eine weitere Stunde bei Raumtemperatur gerührt und anschließend das Pyridiniumhydrochlorid abfiltriert. Das gelbe Filtrat wurde unter mehrmaligem Codestillieren mit Toluol im Vakuum eingeengt- Der so gewonnene orangefarbene Sirup wurde in Dichlormethan/Toluol (1:1) aufgenommen, zweimal mit 1 M HCl-Lösung gewaschen und jeweils viermal mit gesättigter (10%iger) Natriumhydrogencarbonatlösung extrahiert. Die vereinten wässrigen Oberphasen wurden bis zur Trübung der Lösung mit konz. Salzsäure versetzt. Die so entstandene wässrige suspension wurde dreimal mit Dichlormethan unter Aufnahme des ausgefallenen Feststoffes extrahiert. Die vereinigten organische Extrakte wurden über Na₂SO₄ getrocknet und unter Erhalt einen gelben Feststoffes im Vakuum eingeengt.
Ausbeute: 86 g (72,1 %).

Umkristallisieren dieses farbigen rohen Feststoffes aus wenig warmem Dichlormethan sowie Suspendieren des Kristallisats in wenig kaltem Essigester mit anschließender Filtration lieferte ein weißes Kristallisat.
Ausbeute nach Umkristallisation: 47.2 g (39.5 %)
¹³C-NMR: δ = 166,21 (CO-Ascorbyl), 155,13 (CO-Carbonat), 153,46 (C-2-Ascorbyl), 114,79 (C-3-Ascorbyl), 110,71 (C(CH₃)₂), 76,03 (C-4-Ascarbyl), 74,74 (CH(CH₃)₂, 73,52 (C-5-Aacorbyl). 65,29 (C-6-Ascorbyl), 25.78, 25,55 (C(CH₃)₂), 21,51, 21,50 (CH(CH₃)₂) ppm.

### Beispiel 4

### 2-O-(Isopropyloxycarbonyl)-L-ascorbinsaure

47 g (0,155 mol) 5,6-O-Iaopropyliden-2-O-(isopropyloxycarbonoyl)-L-ascorbinsäure wurden 12 Stunden bei Raumtemperatur in 200 ml eines Gemisches Trifluoressigsäure/Wasser (1:4) gerührt. Die farblose Reaktionslösung wurde unter mehrmaligem Codestillieren im Vakuum eingeengt. Es wurde ein hellbrauner Sirup (56,4 g, 138,3 %) erhalten, der an Kieselgel gereinigt wurde (Laufmittel Dichlormethan/Methanol 3:1).
Ausbeute: 43 g (92 %)
¹H-NMR: δ = 4,81 (H-4), 4,77 (OCH(CH₁)₂), 3,77 (H-5), 3,49 (2x H-6), 1,30-1,25 (OCH(CH₃)₂) ppm.

### Kosmetische Zubereitungen

### Beispiel 5

### Zusammensetzung für fettfreies Sonnenschutz-Gel

### Massengehalt

### (Gew.-%)

| | |
|---|---|
| 0,40 | Acrylate/C₁₀-C₃₀ Alkylacrylate crosspolymer |
| 0,25 | Hydroxyethyl Cellulose |
| 8,00 | Methoxycinnamic-octylester |
| 1,00 | 4-Methylbenzylidene Camphor |
| 0,50 | 6-O-Palmitoyl-2-O-(isoprapyloxycarbonoyl)-L-ascorbinsäure |
| 0,20 | Disodium EDTA |
| 5,00 | Glycerin |
| 0,15 | Fragrance |
| 0,30 | imidamolidinyl urea |
| 0,25 | Sodium methylparaben |
| 0,15 | Sodium propylparaben |
| 5,00 | PEG-25 PABA |
| 0,10 | Sodiumhydroxide |
| ad 100 | Water |

### Beispiel 6

### Zusammensetzung für Feuchtigkeitscreme

### Massengehalt

### (Gew.-%)

| | |
|---|---|
| 6,00 | PEG-7-Hydrogenated castor oil |
| 5,00 | Isopropylpalmitate |
| 6,00 | Mineral oil |
| 5,00 | Jojoba oil |
| 5,00 | Almond oil |
| 0,50 | Tocopherylacetate |
| 2,00 | 6-O-Palmitoyl-2-O-(isopropyloxycarbonoyl)-L-ascorbinsäure |
| 0,60 | Magnesiumstearate |
| 2,00 | PEG-45 / Dodecyl glycol copolymer |
| 5,00 | Glycerin |
| 0,25 | Methylparaben |
| 0,15 | Propylparaben |
| 5,00 | Imidazolidinyl urea |
| 0,15 | Fragrance |
| 0,20 | Disodium EDTA |
| ad 100 | Water |

### Beispiel 7

### Zusammensetzung für Feuchtigkeitscreme

### Massengehalt

### (Gew.-%)

| | |
|---|---|
| 6,00 | PEC-7-Hydrogrenated castor oil |
| 5,00 | Isopropylpalmitate |
| 6,00 | Mineral oil |
| 5,00 | Jojoba oil |
| 5,00 | Almond oil |
| 0,50 | Tocopherylacetate |
| 2,00 | 5,6-O-Isopropyliden-2-O-(isopropyloxycarbonoyl)-L- |
| | ascorbinsäure |
| 0,60 | Magnesiumstearate |
| 2,00 | PEG-45 / Dodecyl glycol copolymer |
| 5,00 | Glycerin |
| 0,25 | Methylparaben |
| 0,15 | Propylparaben |
| 5,00 | Imidazolidinyl urea |
| 0,15 | Fragrance |
| 0,20 | Disodium EDTA |
| ad 100 | Water |

### Beispiel 8

### Zusammensetzung für Nachtcreme ohne Konservierungsmittel

### Massengehalt

### (Gew.-%)

| | |
|---|---|
| 5,00 | PEG-7-Hydrogenated castor oil |
| 4,00 | isopropylpalmitate |
| 4,00 | Caprylic acid/Capriate triglyceride |
| 3,00 | 6-O-Palmitoyl -2-O-(isopropyloxyearbonoyl)-L-ascorbinsäure |
| 1,50 | PEG-45/Podecyl glycol copolymer |
| 0,50 | Magnesiumstearate |
| 1,50 | Dimethicon |
| 4,00 | 1,2 Propyleneglycol |
| 4,00 | Glycerin |
| 8,00 | 611 Alcohol |
| 2,00 | Kollagen |
| 0,15 | Fragrance |
| ad 100 | Water |

### Beispiel 9

### Zusammensetzung für Antifaltencreme

### Massengehalt

### (Gew.-%)

| | |
|---|---|
| 6,00 | PEG-7-Hydrogenated castor oil |
| 5,00 | Isopropylpalmitate |
| 10,00 | Mineral oil |
| 3,00 | Caprylic acid/Capriate triglyceride |
| 0,60 | Magnesiumstearate |
| 1,00 | 6-o-Palmitoyl-2-o-(isopropyloxycarbonoyl)-L-ascorbinsäure |
| 1,50 | Tocopherylacetate |
| 2,00 | PEG-45/Dodecyl Glycol Copolymer |
| 0,05 | Tocopherol |
| 0,20 | Retinol |
| 0,30 | Glycerin |
| 0,70 | Magnesiumsulfate |
| 0,25 | Methylparaben |
| 0,15 | Propylparaben |
| 0,20 | Sodiutnascorbylmonophosphat |
| 0,10 | α-Tocopherol |
| 0,10 | Ascorbylpalmitate |
| 0,15 | Fragrance |
| ad 100 | Water |

### Beispiel 10

### Zusammensetzung für Antifaltencreme

### Massengehalt

### (Gew.-t)

| | |
|---|---|
| 6,00 | PEG-7-Hydrogenated castor oil |
| 5,00 | Isopropylpalmitate |
| 10,00 | Mineral oil |
| 3,00 | Caprylic acid/Capriate triglyceride |
| 0,60 | Magnesiumstearate |
| 1,00 | 2,5,6-Tri-O-(isopropyloxycarbonyl)-L-ascorbinsäure |
| 1,50 | Tocopherylacetate |
| 2,00 | PEG-45/Dodecyl Glycol Copolymer |
| 0,05 | Tocopherol |
| 0,20 | Retinol |
| 0,30 | Glycerin |
| 0,70 | Magnesiumsulfate |
| 0,25 | Methylparaben |
| 0,15 | Propylparaben |
| 0,20 | Sodiumascorbylmonophosphat |
| 0,10 | α-Tocopherol |
| 0,10 | Ascorbylpalmitate |
| 0,15 | Fragrance |
| ad 100 | Water |

### Beispiel 11

### Zusammensetzung für Feuchtigkeits Tagescreme

### Massengehalt

### (Gew.-%)

| | |
|---|---|
| 2,00 | Ceteareth/6 |
| 2,00 | ceteareth/25 |
| 10,00 | Mineral oil |
| 3,00 | Caprylic acid/Capriate triglyceride |
| 3,00 | Isostearic acid |
| 3,00 | 6-O-Palmitoyl-2-O-(isopropyloxycarbonoyl)-L-ascorbirrsäure |
| 1,50 | Tocopherylacetate |
| 2,00 | D-Panthenol USP |
| 0,05 | Tocopherol |
| 0,20 | Retinol |
| 0,30 | Glycerin |
| 0,15 | Dibromocyanobutane |
| 0,20 | Sodiumascorbylmonophosphate |
| 0,10 | α-Tocopherol |
| 0,10 | Ascorbylpalmitate |
| 0,15 | Fragrance |
| ad 100 | Water |

### Beispiel 12

### Zusammensetzung für Feuchtigkeits Tagescreme

### Massengehalt

### (Gew.-%)

| | |
|---|---|
| 2,00 | Ceteareth/6 |
| 2,00 | Ceteareth/25 |
| 10,00 | Mineral oil |
| 3,00 | Caprylic acid/Capriate triglyceride |
| 3,00 | Isostearic acid |
| 3,00 | 2-O-(isopropyloxycarbonoyl)-L- ascorbinsäure |
| 1,50 | Tocopherylacetate |
| 2,00 | D-Panthenol USP |
| 0,05 | Tocopherol |
| 0,20 | Retinol |
| 0,30 | Glycerin |
| 0,15 | Dibromocyanobutane |
| 0,20 | Sodiumascorbylmonophosphate |
| 0,10 | α-Tocopherol |
| 0,10 | Ascorbylpalmitate |
| 0,15 | Fragrance |
| ad 100 | Water |

## Patentansprüche

1. Verwendung von Ascorbinsäurederivaten der allgemeinen Formel I, 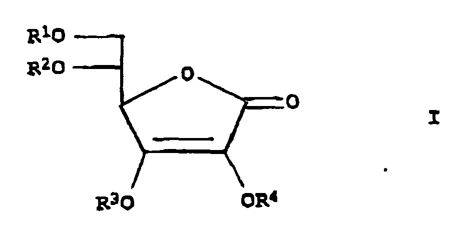 in der die Variablen unabhängig voneinander folgende Bedeutung haben:
R¹ Wasserstoff, C₁-C₂₀-Acyl, C₁-C₆-Alkoxycarbonyl;
R² Wasserstoff, C₁-C₂₀-Acyl, C₁-C₆-Alkoxycarbonyl,
wobei R¹ kein C₁-C₆-Alkoxycarbonyl-Rest sein darf, wenn R² für Wasserstoff steht;
R³ Wasserstoff oder ein Kation, ausgewählt aus der Gruppe, bestehend aus NH₄⁺, Alkali- und Erdalkalimetallkationen;
R⁴ C₁-C₆-Alkoxycarbonyl.
als Antioxidans in kosmetischen Zubereitungen.

2. Kosmetische Zubereitungen, enthaltend 0,01 bis 10 Gew. % einer der Verbindungen der Formel gemäß Anspruch 1 sowie Obliche kosmetische Hilfs- und Zusatzstoffe.

3. Ascorbinsäurederivate der Formel Ia, 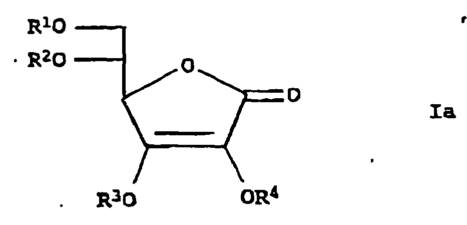 in der die Variablen unabhängig voneinander folgende Bedeutung haben:
R¹ und R² unabhängig voneinander
Wasserstoff oder C₁-C₂₀-Acyl;
R³ Wasserstoff oder ein Kation, ausgewählt aus der Gruppe, bestehend aus NH₄⁺, Alkali- und Erdalkalimetallkationen;
R⁴ C₁-C₆-Alkoxycarbonyl.

4. Verfahren zur Herstellung von Ascorbinsäurederivaten der Formel Ia, **dadurch gekennzeichnet, daß** man ein Ascorbinsäurederivat der Formel IIa, 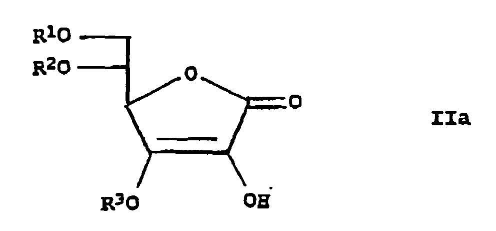 in dem die Substituenten R¹ bis R³ die in Anspruch 3 genannte Bedeutung haben, mit einem Halogenkohlensäurealkylester der Kettenlänge C₁ bis C₆ umsetzt.

5. Verfahren nach Anspruch 4, **dadurch gekennzeichnet, daß** man Chlor- oder Bromkohlensäurealkylester der Kettenlänge C₁ bis C₆ verwendet..

## Claims

1. The use of ascorbic acid derivatives of the formula I 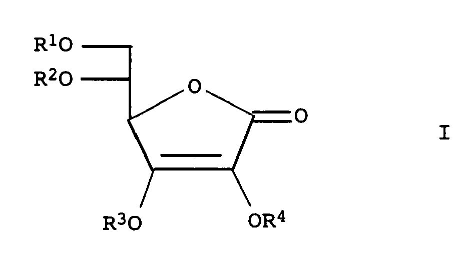 where the variables independently of one another have the following meanings:
R¹ is hydrogen, C₁-C₂₀-acyl, C₁-C₆-alkoxycarbonyl;
R² is hydrogen, C₁-C₂₀-acyl, C₁-C₆-alkoxycarbonyl;
although R¹ must not be a C₁-C₆-alkoxycarbonyl radical when R² is hydrogen;
R³ is hydrogen or a cation selected from the group consisting of NH₄⁺, alkali metal and alkaline earth metal cations;
R⁴ is C₁-C₆-alkoxycarbonyl,
as an antioxidant in cosmetic preparations.

2. A cosmetic preparation comprising from 0.01 to 10% by weight of one of the compounds of the formula I according to claim 1, and customary cosmetic auxiliaries and additives.

3. An ascorbic acid derivative of the formula Ia, 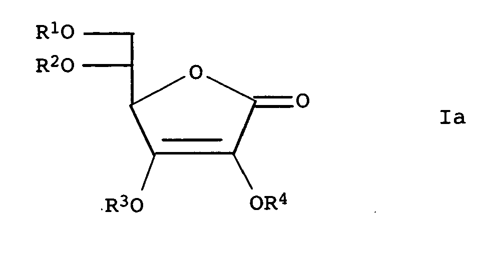 where the variables independently of one another have the following meanings:
R¹ and R² independently of one another
are hydrogen or C₁-C₂₀-acyl;
R³ is hydrogen or a cation selected from the group consisting of NH₄⁺, alkali metal and alkaline earth metal cations;
R⁴ is C₁-C₆-alkoxycarbonyl.

4. A process for preparing ascorbic acid derivatives of the formula Ia, which comprises reacting an ascorbic acid derivative of the formula IIa 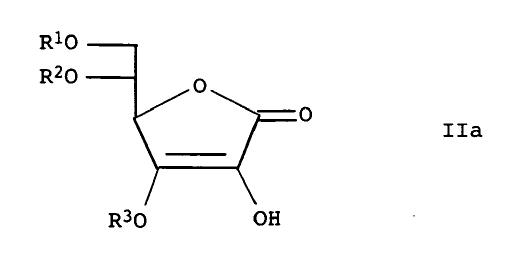 where the substituents R¹ to R³ have the meanings mentioned in claim 3, with an alkyl halocarbonate of chain length C₁ to C₆.

5. The process according to claim 4, which comprises using alkyl chloro- or bromocarbonates of chain length C₁ to C₆.

## Revendications

1. Utilisation de dérivés d'acide ascorbique de formule générale I 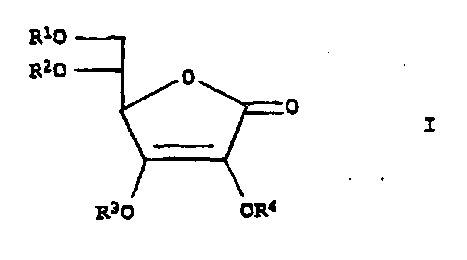 dans laquelle les variables ont indépendamment l'une de l'autre la signification suivante :
R¹ représente l'hydrogène, un acyle en C₁-C₂₀, un alkoxycarbonyle en C₁-C₆;
R² représente l'hydrogène, un acyle en C₁-C₂₀, un alkoxycarbonyle en C₁-C₆,
R¹ ne pouvant être un radical alkoxycarbonyle en C₁-C₆ si R² représente l'hydrogène;
R³ représente l'hydrogène ou un cation sélectionné parmi le groupe constitué du NH₄⁺, des cations de métal alcalin et alcalino-terreux;
R⁴ représente un alkoxycarbonyle en C₁-C₆,
comme antioxydants dans des préparations cosmétiques.

2. Préparations cosmétiques contenant 0,01 à 10% en poids d'un des composés de formule I selon la revendication 1 ainsi que des adjuvants et additifs cosmétiques habituels.

3. Dérivés d'acide ascorbique de formule Ia, 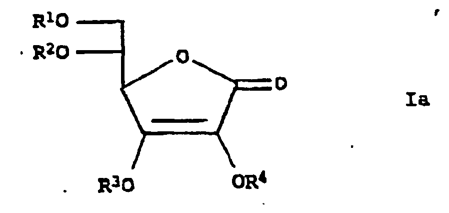 dans laquelle les variables ont indépendamment l'une de l'autre, la signification suivante:
R¹ et R² indépendamment l'un de l'autre,
représentent l'hydrogène ou un acyle en C₁-C₂₀;
R³ représente un hydrogène ou un cation sélectionné parmi le groupe constitué du NH₄⁺, des cations de métal alcalin et alcalino-terreux;
R⁴ représente un alkoxycarbonyle en C₁-C₆.

4. Procédé de préparation de dérivés d'acide ascorbique de formule Ia, **caractérisé en ce qu'**on met à réagir un dérivé d'acide ascorbique de formule IIa, 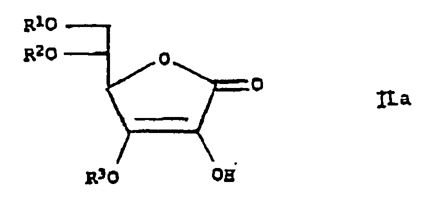 dans lequel les substituants R¹ à R³ ont la signification donnée à la revendication 3, avec un alkylester d'acide halogénocarbonique d'une longueur de chaîne C₁ à C₆.

5. Procédé selon la revendication 4, **caractérisé en ce qu'**on utilise un alkylester d'acide chloro- ou bromocarbonique d'une longueur de chaîne C₁ à C₆.
